# EUROPEAN PATENT SPECIFICATION

(11) **EP 3 125 953 B1**
(45) Date of publication and mention of the grant of the patent: **06.06.2018**
(21) Application number: 15713962.7
(22) Date of filing: 02.04.2015
(51) Int. Cl.: A61L 2/26, B65B 43/22

(54) **STERILIZING MACHINE FOR LOOSE PRODUCTS**
STERILISIERMASCHINE FÜR LOSE PRODUKTE
MACHINE DE STÉRILISATION POUR PRODUITS EN VRAC

(30) Priority: 04.04.2014 IT UD20140060
(43) Date of publication of application: 08.02.2017
(73) Proprietor: Icos Pharma S.p.A., 33080 Zoppola (IT)
(72) Inventor: ZARDINI, Fabio, 31033 Castelfranco Veneto (IT)
(74) Representative: Petraz, Gilberto Luigi
(86) International application number: PCT/EP2015/097001
(87) International publication number: WO 2015/150581

(56) References cited:
- EP-A2- 0 836 991
- CN-A- 101 837 845
- US-A- 5 238 660
- US-A1- 2013 318 914

## Description

### FIELD OF THE INVENTION

Forms of embodiment described here concern a sterilizing machine for loose products, usable for example in hospitals and the pharmaceutical and food sector, or in the industrial field for the production of said loose products.

In particular, forms of embodiment described here concern a sterilizing machine able to sterilize the loose products in a rotary drum, which loose products can be for example closing stoppers for test tubes for laboratory analyses, rubber stoppers for bottles containing pharmaceutical products, plungers for sterilized, disposable syringes or metal capsules for sealing pharmaceutical containers.

In particular, forms of embodiment described here concern a sterilizing machine which automatically discharges the loose products once sterilized.

### BACKGROUND OF THE INVENTION

It is known that in hospitals or the pharmaceutical sector or other fields, for example the food industry, it is necessary to use sterile products to prevent contamination or alterations in the substances with which they come into contact, or to prevent the introduction of bacteria, germs, other corpuscles or potentially pathogenous or harmful agents into environments where total absence thereof is required.

Among such sterile products, in all the fields mentioned above, loose products are normally included, also small-size, such as for example stoppers for closing test tubes intended to contain substances to be subjected to laboratory analysis, or small instruments or objects used in operating theaters or laboratories.

These loose products can already be sterile at the moment of production, and sold in packs which are also sterile, or they are sterilized before each use and reused several times during their working life.

It is known to sterilize such loose products in sterilizing machines provided with an airtight chamber in which a rotary drum receives the products to be sterilized on a loading side, or so-called "dirty side"; it then sterilizes them during a treatment cycle normally comprised between 1 and 5 hours, and then discharges them on a discharge side, or so-called "sterile side", where they are generally packed in sterile packs. A sterilizing machine of this type is known for example from IT-B-1.268.399.

Rotary drums are known, partitioned into sectors, typically from four to twenty four, each containing a desired quantity of loose products to be delivered into the individual packs after sterilization.

In order to be considered sterile, according to legal regulations, the discharge side must be almost or totally lacking in particles, bacteria, viruses, even of submicronic size. Consequently, the sterilization process must also be configured to prevent, to the utmost degree, the possibility of such particles being present on the discharge side.

Sterilizing machines are known, provided with a discharge apparatus for the loose products, comprising one or more manual drive devices, in which the discharge of the loose products after sterilization includes one or more operations performed manually by an operator present in the discharge zone, on the sterile side of the sterilizing machine.

These operations can include the handling and positioning at the discharge apparatus of the sterile packs, generally bags, in which the sterilized objects are inserted by gravity; moving the discharge apparatus near to the airtight chamber to house the sterilized objects; activating one or more discharge steps as a function of the manual operations being performed; controlling and verifying that the discharge operations are correct.

One disadvantage of known sterilizing machines is that they do not guarantee that the sterile condition continues in the long term, or that submicronic particles are absent from the sterile side of the machine where there is the operator.

Another disadvantage of known sterilizing machines is that they require long and complex operations to prepare the operator, in order to give him an overall sterile condition; such preparation must be accompanied by a long and expensive training for the operator himself.

Furthermore, another disadvantage of known sterilizing machines, which require the presence of an operator, is that often it takes a long time to discharge the loose products from the airtight chamber, in particular due to the times required for handling and positioning the packs which have to accommodate them.

Document US-A-5,238,660 is known, which describes an apparatus for cleaning and sterilizing objects such as pharmaceutical stoppers or caps.

Document CN-A-101837845 is also known, which describes a machine for automatically packaging bags.

There is therefore a need to perfect a sterilizing machine for loose products which can overcome at least one of the disadvantages of the state of the art.

In particular, one purpose of the present invention is to obtain a sterilizing machine for loose products which is able to guarantee the discharge side is maintained sterile as time passes and as sterilization cycles continue.

Another purpose of the present invention is to obtain a sterilizing machine for loose products which allows to speed up the discharge step of the products contained in the airtight chamber and which is reliable and quick in controlling that the discharge step is performed correctly.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, forms of embodiment concern a sterilizing machine for loose products. The sterilizing machine comprises at least a treatment chamber for sterilizing loose products, such as for example small products, for example closing stoppers for test tubes for laboratory analyses, rubber stoppers for bottles containing pharmaceutical products, plungers for sterilized, disposable syringes or metal capsules for sealing pharmaceutical containers.

According to some forms of embodiment, the sterilizing machine comprises an apparatus for automatic packaging into bags of the sterilized loose products exiting from a discharge aperture of the treatment chamber.

The automatic packaging apparatus comprises:
- at least one bag support element positionable at the discharge aperture;
- at least a bag feed device provided with a bag storage chamber and a motorized bag transfer unit to transfer the bags from the bag storage chamber to the bag support element;
- at least one bag opening device to open a bag supported by the bag support element and to allow the bag to receive sterilized loose products exiting from the discharge aperture and a bag sealing device to seal a bag filled with the sterilized loose products.

In this way we obtain the advantage of making the operations to discharge and pack sterilized loose products automatic and therefore quicker.

Another advantage of the present invention is to reduce the probabilities of contamination of the discharge and packaging zone, which is conventionally a sterile environment, due to the presence of the operator.

According to another form of embodiment, the motorized bag transfer unit comprises at least a motorized bag removal element, inside the bag storage chamber, in constant contact with a bag and able to be selectively activated to remove the bag from the bag storage chamber and to transfer it to the bag support element.

According to another form of embodiment, the at least one bag feed device comprises a guide element associated with a bag passage aperture of the bag storage chamber and configured to guide toward the bag support element a bag exiting from the bag passage aperture.

According to another form of embodiment, the automatic packaging apparatus comprises at least a conveying device provided with at least a frame to which one or more deflector members are connected. In a variant of this form of embodiment, the frame comprises a hinging side and is rotatable with respect to the hinging side to assume a lowered position, in which the one or more deflector members cooperate with a respective one of the bags. In another variant of this form of embodiment, the conveying device comprises linear actuators connected to the frame to translate the frame to a lowered position, in which the one or more deflector members cooperate with a respective one of the bags.

According to another form of embodiment, the bag support element comprises a pair of mobile plates. Furthermore, in this form of embodiment, the automatic packaging apparatus comprises at least a lower movement device and an upper movement device to respectively move, reciprocally closer and away from each other, corresponding lower edges and upper edges of one of the plates with respect to the other of the plates.

According to another form of embodiment, the automatic packaging apparatus comprises an automatic discharge device, configured to cooperate with the at least one bag support element to receive and discharge the bag from the at least one bag support element.

Other forms of embodiment described here concern a method for packaging into bags loose products sterilized in a treatment chamber of a sterilizing machine according to the present disclosure. According to one form of embodiment, the method comprises a step of preparing the bags, which provides to transfer automatically each bag from a bag storage chamber to a bag support element and subsequently to position each bag in an open condition in correspondence to a discharge aperture of the treatment chamber in order to receive therefrom the loose products, and a step of filling and closing the bags, which provides to fill the bags with dosed quantities of the loose products exiting from the discharge aperture and to seal the bags after filling them.

According to another form of embodiment, after sealing, the method provides to automatically discharge the bags by transferring them from the bag support element to an automatic discharge device, and subsequently distancing them from the support element.

According to another form of embodiment, after opening the bag, the method provides to automatically verify that the bag has actually been correctly positioned in the bag support element, and to position the bag in correspondence with the corresponding discharge aperture following a positive outcome of the verification. In a variant of this form of embodiment, following a positive outcome of the verification it is provided to support and clamp the bag stably in its position in correspondence with the corresponding discharge aperture.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a three-dimensional view of a sterilizing machine according to forms of embodiment described here;
- fig. 2 is a plan view of fig. 1;
- fig. 3 is a lateral view of fig. 2;
- fig. 4 is a section view from IV to IV of fig. 3;
- fig. 5 is a section view from V to V of fig. 3;
- fig. 6 is a lateral view of a component of the machine in fig. 1;
- fig. 7 is a plan view of the component in fig. 6;
- fig. 8 is an exploded three-dimensional view of the component in fig. 6;
- fig. 9 is an exploded three-dimensional view of another component of the machine in fig. 1;
- fig. 10 is a partial lateral view of the component in fig. 9, in two operating positions;
- fig. 11 is a front view of the component in fig. 9;
- fig. 12a is a schematized front view of some forms of embodiment of an automatic packaging apparatus for loose products comprised in the machine in fig. 1;
- fig. 12b is a variant of fig. 12a;
- figs. 13 to 20 show different operating conditions of an automatic packaging apparatus according to forms of embodiment described here during some packaging steps;
- fig. 21 is a schematic front view of other forms of embodiment of an automatic packaging apparatus for loose products, comprised in the machine in fig. 1.

In the following description, the same reference numbers indicate identical parts of the sterilizing machine according to the present invention, also in different forms of embodiment. It is understood that elements and characteristics of one form of embodiment can be conveniently incorporated into other forms of embodiment without further clarifications.

### DETAILED DESCRIPTION OF SOME FORMS OF EMBODIMENT

We shall now refer in detail to the various forms of embodiment of the present invention, of which one or more examples are shown in the attached drawings. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

Before describing these forms of embodiment, we must also clarify that the present description is not limited in its application to details of the construction and disposition of the components as described in the following description using the attached drawings. The present description can provide other forms of embodiment and can be obtained or executed in various other ways.

We must also clarify that the phraseology and terminology used here is for the purposes of description only, and cannot be considered as limitative. The use of terms such as "including", "comprising", "having" and their variations is intended to include the elements listed after them and their equivalents, and also additional elements. Unless otherwise specified, terms such as "mounted", "connected", "supported" and "coupled" and their variations are used in the widest sense and include both direct and indirect assemblies, connections, supports and couplings. Furthermore, the terms "connected" and "coupled" cannot be limited to physical or mechanical connections or couplings.

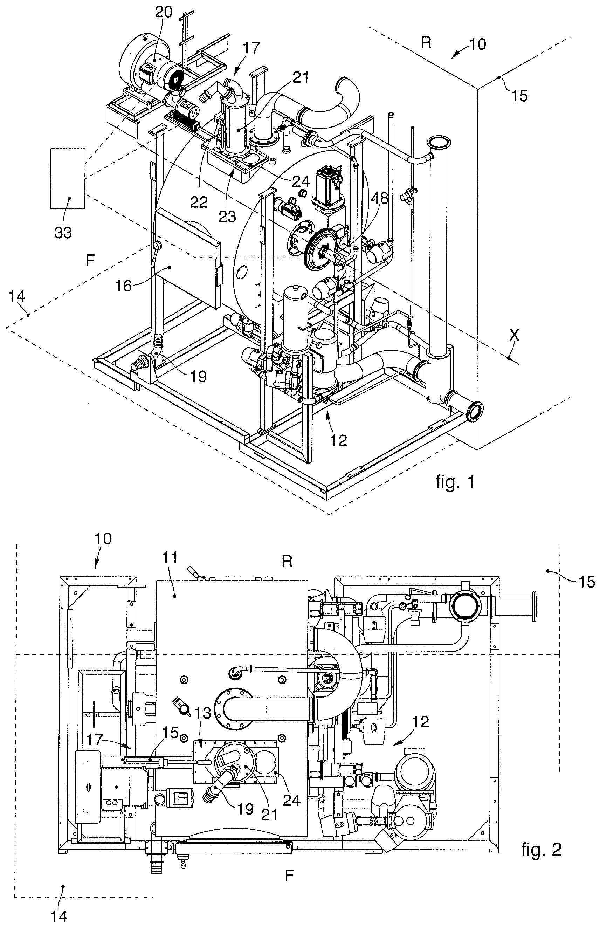

Figs. 1 to 5 are used to describe example forms of embodiment of a sterilizing machine 10 used to sterilize loose products, for example small or medium sized, such as for example small instruments for clinical, hospital or food use or in general laboratory instruments, or closing stoppers for test tubes, rubber stoppers for bottles containing pharmaceutical products, plungers for sterilized, disposable syringes or metal capsules for sealing pharmaceutical containers.

For reasons of convenience the sterilizing machine 10 in figs. 1 to 5 is shown without its external casing which conventionally encloses, supports and protects the internal components.

In some forms of embodiment, which can be combined with all the forms of embodiment described here, the sterilizing machine 10 comprises a treatment chamber 11 in which the loose products are sterilized, and a system of auxiliary plants 12 cooperating with the treatment chamber 11 to create inside it the process conditions needed for sterilization.

The process conditions can provide specific heat or thermo-hygrometric conditions, obtained for example by administering, in the treatment chamber 11, heat and/or steam at a desired temperature, chemical conditions, obtained for example by inserting into the treatment chamber 11 a determinate quantity of chemical agents functional to sterilization, pressure conditions, for example subjecting the treatment chamber 11 to vacuum.

In some forms of embodiment, which can be combined with all the forms of embodiment described here, the treatment chamber 11 contains a rotary drum 13 in which, in turn, the loose products are contained during sterilization. The rotary drum 13 is mounted on a shaft 48 rotating around an axis of rotation X.

In some forms of embodiment, which can be combined with all the forms of embodiment described here, the sterilizing machine 10 includes a loading zone 14, for loading the loose products into the rotary drum 13, and a discharge zone 15 for the loose products after sterilization, for example positioned on the opposite side of the sterilizing machine 10 with respect to the loading zone 14.

In particular, the discharge zone 15 can be a sterile chamber, or "clean room", with a controlled atmosphere.

It can be provided that the loading zone 14 is located in a front zone of the sterilizing machine 10, indicated by way of example in the attached drawings by the reference letter F, and that the discharge zone 15 is located in a rear zone of the sterilizing machine 10, indicated by way of example in the attached drawings by the reference letter R.

In some forms of embodiment, which can be combined with all the forms of embodiment described here, the sterilizing machine 10 can include a manual loading aperture 14a provided with a loading door 16, to load the loose products to be sterilized into the treatment chamber 11.

In possible solutions, the treatment chamber 11 can include an automatic loading aperture 14b, in addition to or as an alternative to the manual loading aperture 14a.

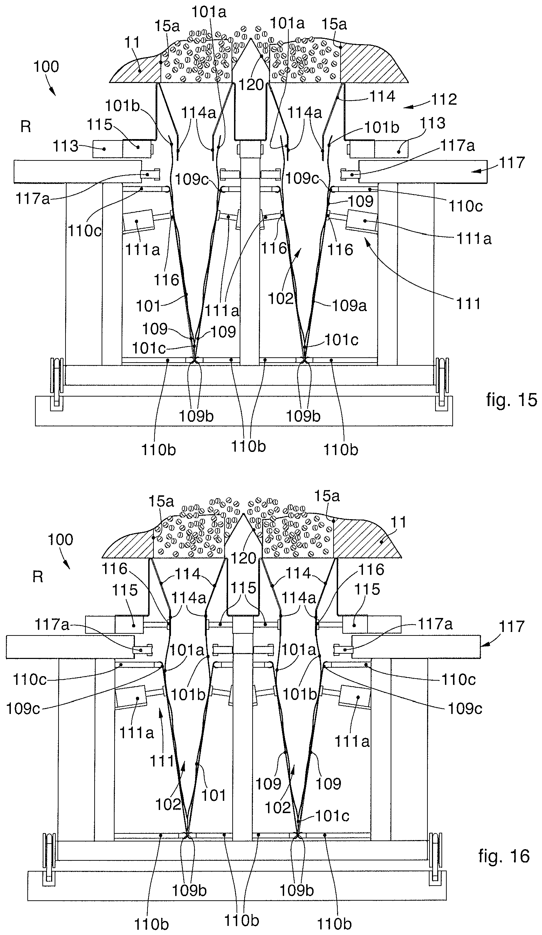

In some forms of embodiment, which can be combined with all the forms of embodiment described here, the treatment chamber 11 can also include one or more discharge apertures 15a, provided in the discharge zone 15 to discharge the sterilized loose products at end-of-cycle. In a possible form of embodiment a single discharge aperture 15a can be provided for example, or two, three or even more than three discharge apertures 15a can be provided. For example, a possible form of embodiment, which can be combined with all the forms of embodiment described here, can provide two discharge apertures 15a. In possible forms of embodiment, which can be combined with all the forms of embodiment described here, the sterilizing machine 10 can be provided with a deflector element 120 (see figs. 15 and 16 for example) disposed in cooperation with the discharge zone 15, upstream of the discharge apertures 15a, and configured to divide into several streams the sterilized mass of loose products to be discharged, for example two streams in the case of two discharge apertures 15a, directed toward respective discharge apertures 15a.

In some forms of embodiment, described by way of example with reference to figs. 1 to 5, the sterilizing machine 10 can include, in its auxiliary plant system 12, an automatic loading apparatus 17 to feed the loose products automatically into the rotary drum 13 inside the treatment chamber 11.

In some forms of embodiment, which can be combined with all the forms of embodiment described here, the automatic loading apparatus 17 can include one or more storage containers 18 for the loose products, one or more suction pipes 19, each connected to a respective container 18 and to a suction device 20, the latter provided to create a depression inside the suction pipes 19 so as to remove from the corresponding container 18 the loose products contained therein.

For example, a single suction pipe 19 can be provided, which removes the loose products from a container 18 due to the effect of the depression created by the suction device 20.

In possible implementations, a dosing receptacle 21 can be provided, connected to the suction pipe 19. The dosing receptacle 21 can receive a dosed quantity of loose products from the suction pipe 19, according to the loading needs of the rotary drum 13.

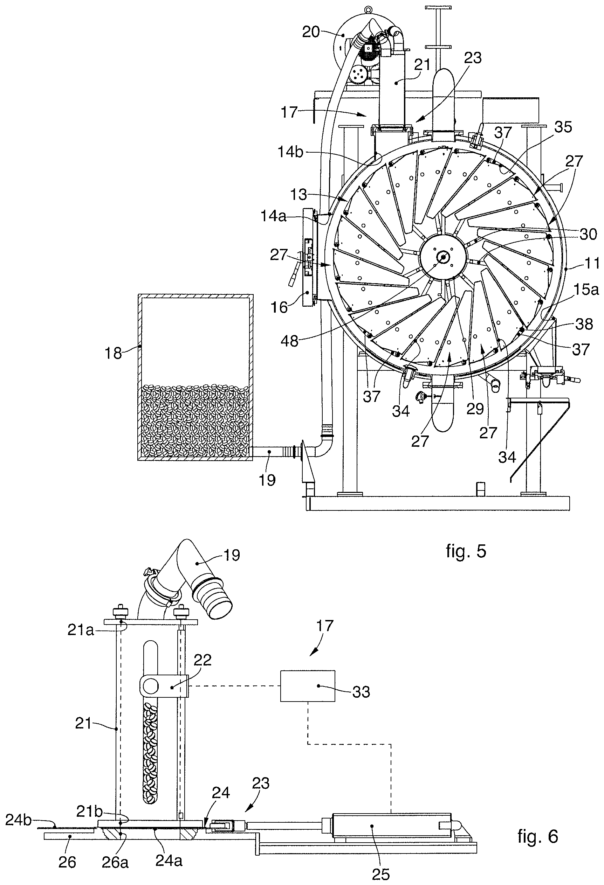

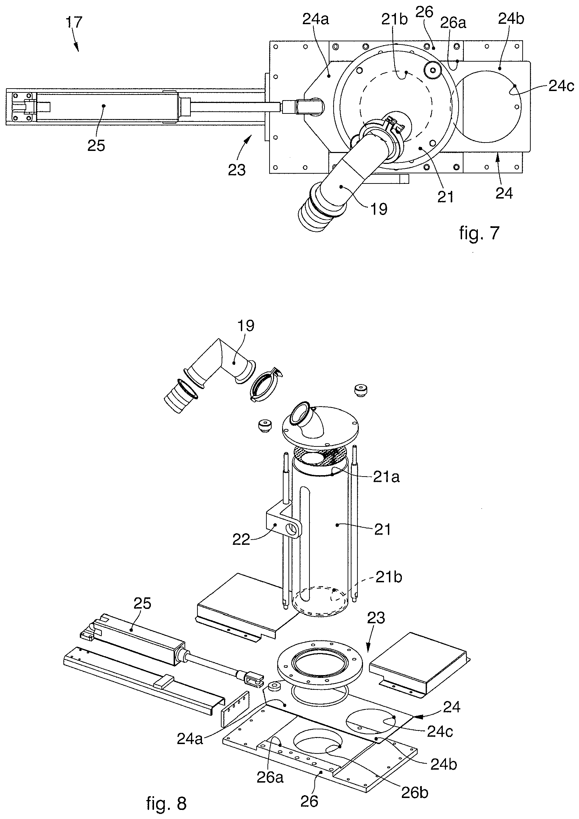

Figs. 6 to 8 are used to described forms of embodiment of the automatic loading apparatus 17, in which it can include an interception device, such as a guillotine valve 23, for example positioned below the dosing receptacle 21, configured to manage, by means of controlled opening and closing, the dosed loading of the loose products inside the rotary drum 13.

In some forms of embodiment, which can be combined with all the forms of embodiment described here, the automatic loading apparatus 17 can include a control sensor 22, which can be provided to control and verify that the dosing receptacle 21 is full. For example, the control sensor 22 can be an optical or gravimetric sensor, or a combination of the two.

In some forms of embodiment, which can be combined with all the forms of embodiment described here, the dosing receptacle 21 can have a tubular shape, and has an entrance aperture 21a communicating with the suction pipe 19 to receive the loose products from the latter.

In some forms of embodiment, which can be combined with all the forms of embodiment described here, the dosing receptacle 21 also includes an exit aperture 21b, through which the loose products exit from the dosing receptacle 21 in a manner controlled by the guillotine valve 23.

The dosing receptacle 21, in some forms of embodiment, can be put in an upper zone of the treatment chamber 11, so as to feed the rotary drum 13, delivering into it its dosed content of loose products due to gravity.

In some forms of embodiment, which can be combined with all the forms of embodiment described here, the guillotine valve 23 can be provided with a mobile plate 24, positioned in correspondence with the exit aperture 21b of the dosing receptacle 21.

In some forms of embodiment, which can be combined with all the forms of embodiment described here, the mobile plate 24 can include a blind portion 24a and a holed portion 24b, in which a through hole 24c is made, with a size coordinated with that of the exit aperture 21b.

In some forms of embodiment, which can be combined with all the forms of embodiment described here, the guillotine valve 23 can also include a linear actuator 25, connected to the mobile plate 24 to move it transversely to the dosing receptacle 21 to selectively position the blind portion 24a or the through hole 24c in correspondence with the exit aperture 21b.

The blind portion 24a of the mobile plate 24 is kept in correspondence with the exit aperture 21b during the filling of the dosing receptacle 21, after which the mobile plate 24 is made to translate to position the through hole 24c in correspondence with the exit aperture 21b, so that the alignment of the exit aperture 21b and the through hole 24c allows the loose products to pass through gravity inside the rotary drum 13.

In possible solutions, the automatic loading apparatus 17 can also include a flange 26, which functions both as a support for the dosing receptacle 21 and also as a guide for the sliding of the mobile plate 24.

In some forms of embodiment, which can be combined with all the forms of embodiment described here, the flange 26 can include a sliding groove 26a, inside which the mobile plate 24 is positioned sliding, and a through hole 26b aligned with the exit aperture 21b of the dosing receptacle 21.

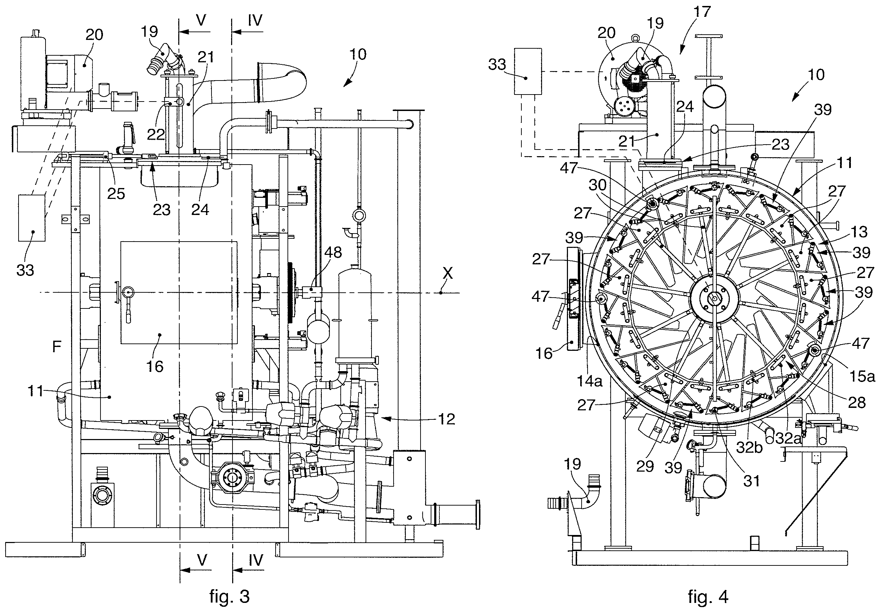

In some forms of embodiment, the rotary drum 13 has a substantially cylindrical shape and is divided into a plurality of containing sectors 27, conventionally from four to twenty four, and which in figs. 4 and 5, by way of example, number twenty.

With reference by way of example to forms of embodiment described using fig. 4, the rotary drum 13 comprises a support frame 28 to which all the containing sectors 27 are attached, in a homogeneous and equidistanced circumferential distribution.

In some forms of embodiment, the support frame 28 can comprise a hub 29, mounted integrated on the shaft 48 to transmit the motion thereof to the rotary drum 13.

In some forms of embodiment, which can be combined with all the forms of embodiment described here, the support frame 28 can also include a plurality of support arms 30, having one end attached to the hub 29 and branching off it radially so as to connect, at its opposite end, to a support ring 31.

In possible implementations, each containing sector 27 is attached to the support frame 28 by removable connection means, such as for example bolts 32a and attachment brackets 32b, provided to connect the containing sectors 27 to the support arms 30 or to the support ring 31.

In some forms of embodiment, each containing sector 27 can include a containing body 34 that can have a pyramid shape, truncated pyramid or conical or truncated cone shape, and externally delimits a loading compartment 35. The volume of the loading compartment 35 identifies the dosed quantity of loose products that can be contained in each containing sector 27.

In some forms of embodiment, which can be combined with all the forms of embodiment described here, the sterilizing machine 10 can include a command and control unit 33 connected to the shaft 48 to command the rotation of the rotary drum 13 and to position each of the containing sectors 27, in sequence, selectively at the manual loading aperture 14a, the automatic loading aperture 14b, or the discharge aperture 15a.

In possible implementations, the command and control unit 33 can be connected to the suction device 20, the linear actuator 25, the control sensor 22 and the rotary shaft 48, to control and command, in a suitably synchronized manner, the functioning of the components of the sterilizing machine 10 in order to effect, automatically, the controlled and dosed loading of the loose products into each loading compartment 35 of the containing sectors 27 of the rotary drum 13.

The division of the rotary drum 13 into containing sectors 27 is functional to the packaging of the loose products at the end of sterilization: in this way, the loose products are already divided and dosed in the desired quantities for packaging.

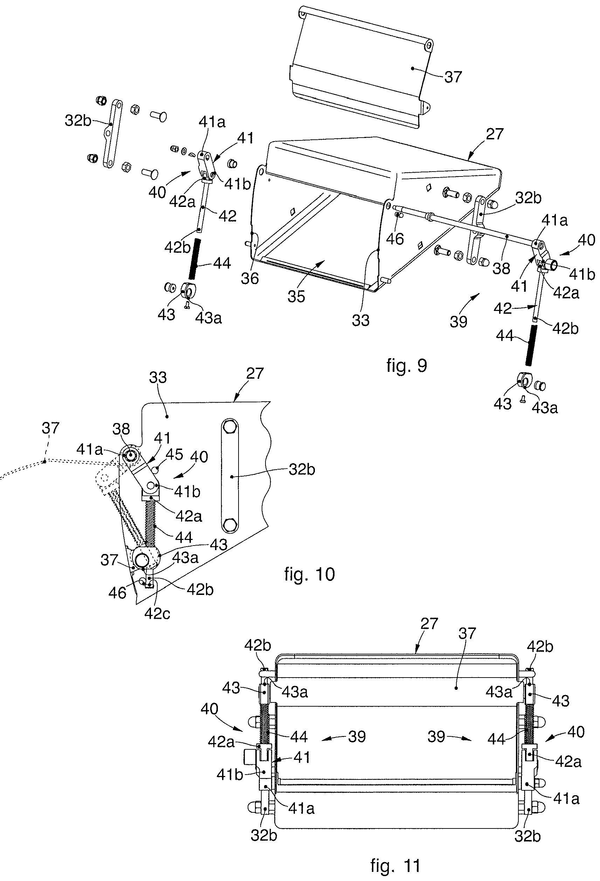

Figs. 9 to 11 are used to describe possible forms of embodiment, which can be combined with all the forms of embodiment described here, of a containing sector 27 where the containing body 34 has a substantially truncated pyramid shape with a quadrilateral base, in correspondence with which a loading door 36 is provided, which puts the loading compartment 35 into communication with the outside of the containing sector 27.

In possible implementations, each containing sector 27 includes a door 37 hinged to the containing body 34, for example by means of a pin 38, and rotatable with respect to the latter to close the loading door 36 during the sterilization process, and to keep the loading door 36 open during the loading and unloading of the loose products.

During use, that is, once attached to the support frame 28, the containing sectors 27 are disposed radially with respect to the shaft 48 and so that the loading door 36 is located in the radially most peripheral zone of the rotary drum 13.

In possible implementations, each containing sector 27 includes an opening device 39 connected to the door 37 to move it from the closed position to the open position with respect to the loading door 36.

In possible solutions, described by way of example in figs. 9 to 11, the opening device 39 can be symmetrical with respect to the center line of the containing body 34, and can comprise two opening units 40 disposed on opposite sides of the loading door 36.

The opening units 40 are both connected to the door 37 to make it selectively assume the closed position, for example shown in figs. 10 and 11, in which the door 37 covers the loading door 36, and said open position, shown by dashes in fig. 9, in which the door 37, open, frees the loading door 36.

In some forms of embodiment, each opening unit 40 can include a handle 41 having a first end 41a connected to the hinging pin 38 of the door 37 and rotatable around the axis of the pin 38.

In possible implementations, the connection between the first end 41a of the handle 41 and the pin 38 is such that with a rotation of the handle 41 there is a corresponding equal rotation of the pin 38 and, consequently of the door 37 hinged to it, so that the rotation of the handle 41 determines the opening and closing of the door 37.

In some forms of embodiment, which can be combined with all the forms of embodiment described here, the handle 41 can include a second, command end 41b, opposite the first end 41a; a rod 42 is connected to the second end 41b, in correspondence with a first terminal portion 42a thereof.

In some forms of embodiment, which can be combined with all the forms of embodiment described here, the opening unit 40 can include a guide bushing 43, mounted rotatable with respect to the containing body 34 with respect to an axis of rotation parallel to the axis of the pin 38, and distanced from the first end 41a of the handle 41 along a lateral delimitation wall of the loading door 36.

In possible implementations, the rod 42 has a second terminal portion 42b, opposite the first terminal portion 42a, inserted transversely in the guide bushing 43, through and sliding inside it, along its longitudinal extension.

The contact between the second terminal portion 42b and the guide bushing 43 allows the rod 42 to transmit motion of the handle 41 to the guide bushing 43.

The opening unit 40, in possible implementations like those described by way of example with reference to figs. 9 to 11, also includes a thrust member, like a helical spring 44, or other similar or comparable linearly extendible element, for example a pneumatic piston, able to constantly exert a thrust force in the direction of longitudinal extension of the rod 42.

In some forms of embodiment, which can be combined with all the forms of embodiment described here, the helical spring 44 is mounted coaxial to the rod 42 and is configured to constantly exert a thrust force both on the guide bushing 43 and also on the second end 41b of the handle 41.

This allows to maintain the reciprocal positioning of the components of the opening unit 40 both when the door 37 is in its open position, and also when it is in its closed position.

In the first case, that is, in the closed position of the door 37, the helical spring 44 keeps the second end 41b of the handle 41 constantly thrust in contact with a first abutment element 45 protruding from the containing body 34, and the second terminal portion 42b of the rod 42 constantly thrust in contact with a second abutment element 46, also protruding from the containing body 34.

This prevents any spontaneous rotation of both the handle 41 and also the guide bushing 43, and also the spontaneous sliding of the rod 42. To modify the position of the opening unit 40, a moment must be applied to the second end 41b of the handle 41 that is sufficient to overcome the thrust force of the helical spring 44.

In some forms of embodiment, which can be combined with all the forms of embodiment described here, the rod 42 is provided, in its terminal portion 42b, with a shoulder 42c, configured to limit the sliding of the rod 42 inside the guide bushing 43.

To this purpose, in possible implementations, the guide bushing 43 can include an abutment surface 43a configured to contact the shoulder 42c of the terminal portion 42b of the rod 42, to prevent the latter from continuing to slide with respect to the guide bushing 43 beyond what is provided in the closed position of the door 37.

In possible forms of embodiment, it may be provided that the passage of the door 37 from the closed to the open position requires a rotation of the handle 41 of about 90° around the axis of the pin 38, to the condition where it exerts a sufficient moment on the second end 41b.

In the open position, the helical spring 44 keeps its thrust force against the guide bushing 43 on one side, and against the second end 41b of the handle 41 on the other side.

In this way, the helical spring 44 not only keeps the door 37 open, but also has the function of keeping the shoulder 42c of the rod 42 in contact with the abutment surface 43a of the guide bushing 43.

In some forms of embodiment, described by way of example with reference to fig. 4, the sterilizing machine 10 includes one or more drive devices 47 associated with the treatment chamber 11 and cooperating with the opening units 40 to drive them, automatically or semi-automatically, and to selectively determine the passage of the doors 37 from the open position to the closed position or vice versa.

In some forms of embodiment, which can be combined with all the forms of embodiment described here, each drive device 47 can include a rotary actuator 47a to which a drive element 47b is made integral, and made to rotate by the rotary actuator 47a.

In some forms of embodiment, which can be combined with all the forms of embodiment described here, in possible solutions, the sterilizing machine 10 can include three drive devices 47, each positioned in correspondence with one of the apertures 14a, 14b and 15a made in the treatment chamber 11.

The drive devices 47 can be selectively activated by an operator, or they can be connected to the command and control unit 33 and activated by it in coordination with the rotation of the rotary drum 13 depending on whether, on each occasion, one of the containing sectors 27 is presented in correspondence with one of the apertures 14a, 14b, 15a.

The activation of the drive device 47 provides to drive the rotary actuator 47a to make the drive element 47b rotate so that it contacts the second end 41b of the handle of the corresponding containing sector 27, to thus activate - opening or closing, depending on the direction of rotation of the drive element 47b - the mechanism defined by the opening unit 39.

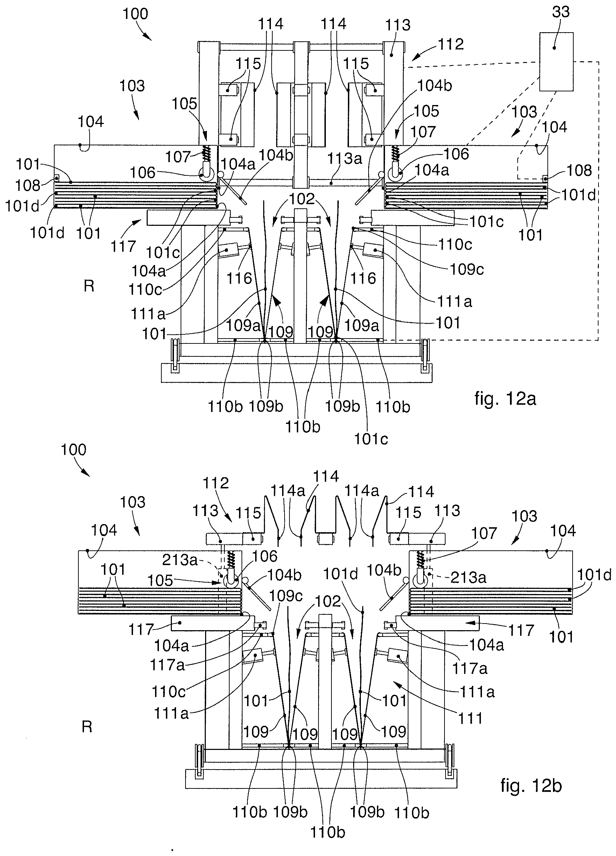

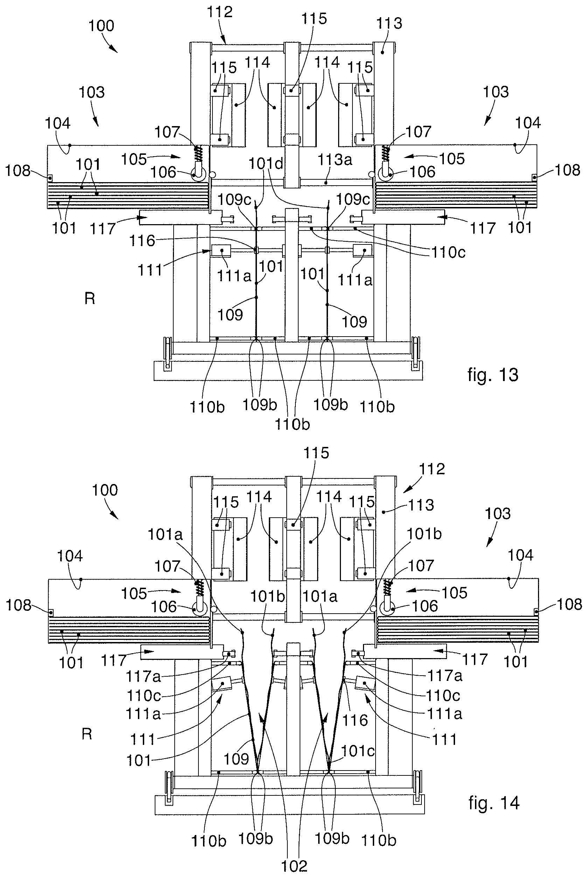

Figs. 12a to 21 are used to describe forms of embodiment, which can be combined with all the forms of embodiment described here, in which the sterilizing machine 10 includes an automatic packaging apparatus 100 positioned in the discharge zone 15.

The automatic packaging apparatus 100 is configured to receive the loose products from the treatment chamber 11 at the end of the sterilization cycle and to automatically pack them into one or more containers, normally sack-shaped, for example bags 101.

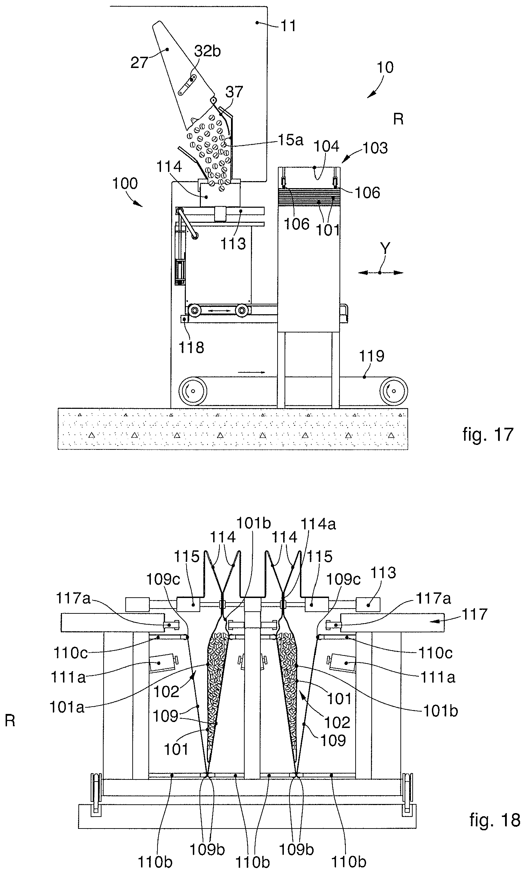

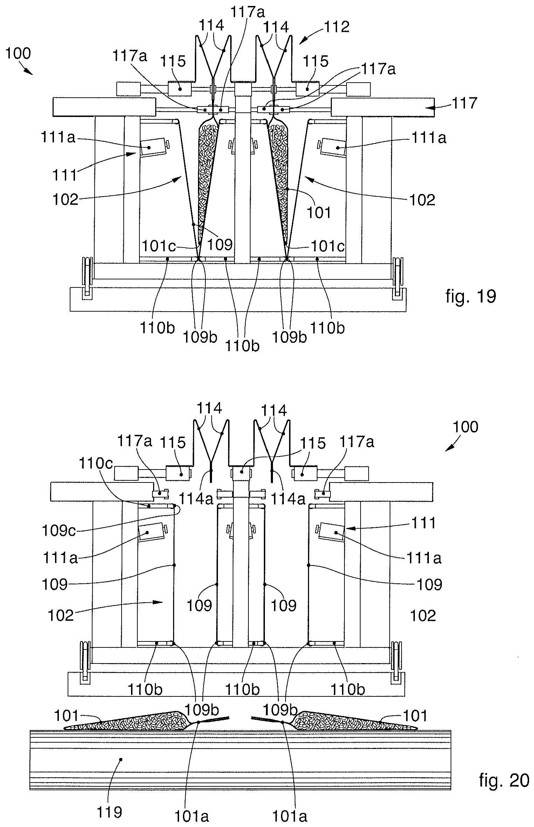

The automatic packaging can provide to prepare and open one or more bags 101, for example one, two, three or more (figs. 12a-15), then to position each of them at a discharge aperture 15a to receive the loose products (figs. 16 and 17), and subsequently to fill the bags 101 with predefined quantities of loose products (fig. 18), and finally to seal the filled bags 101 (fig. 19).

In some forms of embodiment, which can be combined with all the forms of embodiment described here, the bags 101 can each be defined by two sheets 101a, 101b, joined together by welding three of the four perimeter sides and therefore having a closed bottom 101c and an open mouth 101d, opposite the bottom 101c.

The sheets 101a and 101b can be made of one or more polymer or composite materials, monolayer or multicoupled, for example comprising a plurality of polymer, metal and/or paper layers, and can both be made of the same material or of different materials, for example to give the bag 101 different conditions of permeability, opacity, heat, chemical or mechanical resistance.

In some forms of embodiment, the automatic packaging apparatus 100 can also include an automatic discharge device, for example a belt conveyor 119, or an anthropomorphic manipulator or other device to remove the bags 101 configured to remove or receive the bags 101 containing the desired quantities of loose products from bag support elements, or cradles 102, and to discharge them automatically from the discharge zone 15, distancing them from the cradles 102 and from the treatment chamber 11.

In possible solutions, the automatic packaging apparatus 100 can include at least one bag support element or cradle 102, configured to support a bag 101 during a discharge step and during a packaging step, and selectively positionable in correspondence with a discharge aperture 15a of the treatment chamber 11 to allow the bag 101 to receive therefrom the loose products.

In some forms of embodiment, which can be combined with all the forms of embodiment described here, the automatic packaging apparatus 100 can include a bag feed device 103 which, during the bag preparation step, feeds one bag 101 at a time to a cradle 102.

Figs. 12a to 16 and 18 to 20 are used to describe forms of embodiment in which the automatic packaging apparatus 100 includes two cradles 102, adjacent to each other, and two corresponding bag feed devices 103.

This solution is not restrictive of the present invention, since forms of embodiment are possible with a single cradle 102 and a single bag feed device 103, or with more than two cradles 102 and more than two bag feed devices 103, but is described merely by way of example of a possible embodiment of the automatic packaging apparatus 100.

In some forms of embodiment, which can be combined with all the forms of embodiment described here, each bag feed device 103 is provided with a bag storage chamber 104 to temporarily store the bags 101 before they are transferred to the respective cradle 102 for use.

The bags 101 can be stored in the bag storage chambers 104 stacked on top of each other in a closed condition, that is, with the respective two sheets 101a and 101b overlapping.

Each bag storage chamber 104 has an aperture for the passage of the bags 104a, through which the bags 101 exit toward the corresponding cradle 102.

In some solutions, associated with the aperture for the passage of the bags 104a, the bag feed device 103 can include a guide element, for example a plate 104b hinged to the bag storage chamber 104 and which has the function both of closing the bag passage aperture 104a and also of guiding, in this case with its own weight, the bags 101 exiting from the storage chamber 104.

In variant solutions, a profiled element may be provided, or a guide channel, or any other fixed or mobile guide element able to allow the correct passage of the bags 101 from the storage chamber 104 to the corresponding cradle 102.

In some forms of embodiment, which can be combined with all the forms of embodiment described here, one bag 101 can be positioned in the respective bag storage chamber 104 with the bottom 101c facing toward the corresponding bag passage aperture 104a. In this case, two bags 101 can be positioned in the two respective bag storage chambers 104, with the bottom 101c facing toward the corresponding bag passage aperture 104a.

In some forms of embodiment, which can be combined with all the forms of embodiment described here, each bag feed device 103 can also include a motorized bag transfer unit 105, connected to the bag storage chamber 104 and partly inside it, to transfer the bags 101 from the bag storage chamber 104 to a respective cradle 102. In this case, if two bag feed devices 103 are provided, therefore two motorized bag transfer units 105 will also be provided.

In some forms of embodiment, which can be combined with all the forms of embodiment described here, the motorized bag transfer unit 105 can include at least a motorized bag removal element, such as for example a motorized wheel or removal roll 106, located in constant contact with the bags 101 and rotating around an axis parallel to the lying plane of the sheets 101a and 101b inside the bag storage chamber 104.

The lying plane can be horizontal, or inclined toward the cradle 102, to transfer the bags 101 inside it.

The rotation of the motorized removal roll 106 causes a bag 101 to be removed from the bag storage chamber 104 and consequently transferred to the cradle 102 below. In the cradle 102 the bag 101 is inserted with its bottom 101c facing toward the lower part of the cradle 102 and with its mouth 101b facing toward the upper part of the cradle 102, near the treatment chamber 11.

In possible forms of embodiment, which can be combined with all forms of embodiment described here, the motorized bag transfer unit 105 can also include thrust means 107, for example elastic or pneumatic means, connected to a respective motorized removal roll 106 and cooperating with it to keep it constantly in contact with the bag 101 located at the top of the pile of bags 101.

In some forms of embodiment, which can be combined with all the forms of embodiment described here, the thrust means 107 are also configured to thrust the respective motorized removal roll 106 against the top bag 101 to exert thereon a pressure able to guarantee sufficient friction between the motorized removal roll 106 and the top bag 101, in order to move the latter due to the effect of the rotation of the motorized removal roll 106.

In some forms of embodiment, each bag feed device 103 can include a sensor 108, for example to detect the position of the motorized removal roll 106, or an optical measuring device to measure the height of the pile of bags 101, or again a device to measure the position or extension of the thrust means 107, with the function of detecting at least that the bags 101 are nearly finished.

The sensor 108 can signal to the command and control unit 33, continuously during the process, that the bags 101 are nearly finished in the storage chamber 104, and/or that the bags 101 are totally finished, and/or the current level of the bags 101.

Depending on this signal, the command and control unit 33 can possibly command the suspension of the discharge of the loose products from the treatment chamber 11, to allow the bag storage chamber(s) 104 to be refilled.

A cradle 102, as usable in association with the forms of embodiment described here, can include a pair of plates 109 or sheets, which can be made of sterilizable material, such as for example stainless steel.

In some forms of embodiment, which can be combined with all the forms of embodiment described here, the plates 109 are configured mobile and each of them can have lateral edges 109a, a lower edge 109b and an upper edge 109c.

In some forms of embodiment, which can be combined with all the forms of embodiment described here, the automatic packaging apparatus 100 can include a plurality of movement devices, for example one or more lower linear actuators 110b and one or more upper linear actuators 110c, each associated with a lower edge 109b and respectively an upper edge 109c of a corresponding plate 109.

It can also be provided that one or each cradle 102 is served by at least two lower linear actuators 110b and two upper linear actuators 110c.

The linear actuators 110b and 110c can be for example pneumatic pistons, or screw jacks, or again mechanical, magnetic or pneumatic sliders, mobile transversely with respect to the lying plane of the corresponding plate 109.

In particular, the lower linear actuators 110b are configured to move the corresponding lower edges 109b connected to them reciprocally nearer and away, to determine respectively the closing and opening of the lower part of the corresponding cradle 102.

In the same way, the upper linear actuators 110c are configured to move the corresponding upper edges 109c connected to them reciprocally nearer and away, to determine respectively the closing and opening of the upper part of the corresponding cradle 102.

To allow the bags 101 to be transferred into the cradles 102 by the respective bag feed devices 103, the lower linear actuators 110b are configured to close the lower part of the cradles 102, taking into reciprocal contact the lower edges 109b of the plates 109, while the upper linear actuators 110c keep the upper edges 109c of the plates 109 distanced.

Once the bags 101 have been transferred into the cradles 102, the upper linear actuators 110c are driven to take the upper edges 109c of the plates 109 into reciprocal contact as well, completely closing the cradles 102.

In some forms of embodiment, which can be combined with all the forms of embodiment described here, the automatic packaging apparatus 100 includes at least one bag opening device 111 for the automated opening of each bag 101 supported by the cradle 102 and to allow the sterilized loose products to be received.

In some forms of embodiment, which can be combined with all the forms of embodiment described here, the bag opening device 111 includes a pair of holding members 111a for each cradle 102, each of which is positioned near a plate 109 and configured to contact and hold one of the sheets 101a, 101b of the bag 101 contained in the corresponding cradle 102.

Each holding member 111a is configured to hold the respective sheet 101a, 101b by means of adhesion, which can be obtained by creating a depression between the bag 101 and the holding member 111a, or by using sucker-type holding members 111a.

Once the two sheets 101a and 101b of the bag 101 are held, the upper linear actuators 110c are driven to reciprocally distance the upper sides 109c of the plates 109, thus obtaining the opening of the bags 101 in correspondence with their mouths 101d.

In some forms of embodiment, which can be combined with all the forms of embodiment described here, the automatic packaging apparatus 100 also includes a conveying device 112 interposed between the discharge apertures 15a and the cradles 102 to convey into the bags 101 the loose products exiting from the treatment chamber 11.

In some forms of embodiment, described using figs. 12a to 16 and 18 to 20, the automatic packaging apparatus 100 can also include one or more bag sealing devices, for example a heat welding machine 117 provided with welding heads 117a mobile transversely to the bag 101.

In some forms of embodiment, which can be combined with all the forms of embodiment described here, the conveying device 112 can include a frame 113 to which one or more deflector members or tiles 114 are connected, made for example with shaped metal plates.

In some forms of embodiment, which can be combined with all the forms of embodiment described here, the frame 113 is configured mobile with respect to the one or more cradles 102 to position the tiles 114 in a first raised position, in which they are outside the bulk of the bags 101, and a second lowered position, in which at least one wall 114a of each tile 114 cooperates with a bag 101, in particular it interferes with the mouth 101d of a corresponding bag 101.

In some forms of embodiment, described with reference to figs. 12a, 13 and 14, the frame 113 can include a hinging side 113a, for example horizontal, and can be rotatable with respect to it in order to selectively move the tiles 114 nearer to or away from the cradles 102.

In other forms of embodiment, described by way of example using fig. 12b, the conveying device 112 can include linear actuators 213a connected to the frame 113 to translate it nearer to or away from the cradles 102, for example vertically.

The conveying device 112 can include a plurality of presser members 115, for example pneumatic pistons, or screw or spring actuators, mobile transversely to the frame 113, selectively nearer to or away from the tiles 114.

With reference to figs. 12a to 14, for example two presser members 115 can be provided for each tile 114.

Figs. 12a to 19 are used to describe forms of embodiment, which can be combined with other forms of embodiment described here, in which each presser member 115 is configured to assume a first retracted position, in which it is distanced from the corresponding tile 114, and a second thrust position, in which it presses against the wall 114a of the tile 114.

In particular, with reference to figs. 12a to 15, each presser member 115 is located in the first retracted position when the frame 113 is in the raised position, and during the movement of the frame 113 to and from the lowered position.

Figs. 16 to 19 are used to describe by way of example possible forms of embodiment of the presser members 115 in the second thrust position, after the positioning of the frame 113 in the lowered position, to press, against the wall 114a of the corresponding tile 114, one of the sheets 101a or 101b of the bag 101 with which the tile 114 itself interferes.

In this way it is possible to support the bag 101, clamping it in position by means of the pressure exerted by the presser members 115 against the wall 114a of the tile 114, which allows to prevent the bag 101 from collapsing due to the effect of the weight of the objects that are discharged into it.

In possible implementations, the presser members 115 can be provided with bag detection sensors 116, for example defined by switches of the capacitive, optical, electromagnetic, or electric contact type, and configured to verify that the bags 101 are actually and correctly located between the presser members 115 and the walls 114a of the tiles 114.

Fig. 17 is used to describe forms of embodiment, which can be combined with all the forms of embodiment described here, in which the apparatus 100 is configured at least partly translatable in a direction of movement Y from a bag preparation station, distanced from the exit apertures 15a, to a bag filling station in which the bags 101 are put in correspondence with the exit apertures 15a.

Forms of embodiment described here concern a method for packaging into bags 101 loose products sterilized in a treatment chamber 11 of a sterilizing machine 10 according to the present disclosure, which method comprises:
- a step of preparing the bags, which provides to transfer each bag 101 automatically from a bag storage chamber 104 to a bag support element, or cradle, 102 and subsequently to position each of the bags 101 in an open condition in correspondence to a discharge aperture 15a of the treatment chamber 11 in order to receive the loose products,
- a step of filling and closing the bags, which provides to fill the bags 101 with dosed quantities of the loose products exiting from the discharge aperture 15a and to seal the bags 101 after filling them.

In possible implementations, in said bag preparation station, described with reference to forms of embodiment using fig. 17, the bags 101 are positioned in the one or more cradles 102 by the bag feed device 103, opened by the bag opening device 111, and are firmly supported by the conveying device 112, clamping the mouths 101d against the tiles 114.

Afterward, the automatic packaging apparatus 100 is translated to the treatment chamber 11, in the direction of movement Y, possibly keeping the position of the bag feed device 103 fixed.

This translation and the above operations define possible executive implementations of the step of preparing the bags according to the method for packaging the loose products in the bags 101.

Once it has translated to the bag filling station, the automatic packaging apparatus 100 has each bag 101 in correspondence with a discharge aperture 15a of the treatment chamber 11.

One or more position sensors 118, for example optical, magnetic, electromagnetic, or other type suitable to identify the position of the automatic packaging apparatus 100, can be provided to detect and communicate to the command and control unit 33 the position of the automatic packaging apparatus 100, for example with respect to the treatment chamber 11. For example, the one or more position sensors 118 can be positioned on the automatic packaging apparatus 100 and/or on an external surface or wall of the treatment chamber 11.

In some forms of embodiment, which can be combined with all the forms of embodiment described here, the command and control unit 33 can be configured to activate the opening of the doors 37 of the containing sectors 27 positioned in correspondence with the discharge apertures 15a only after it has received a signal that the automatic packaging apparatus 100 is positioned in the bag filling station.

Once each door 37 has been opened, the loose products fall due to gravity into the bag 101 below, filling it according to the predefined dosed quantity, in accordance with possible implementations of said bag filling and closing step.

Some forms of embodiment, which can be combined with all the forms of embodiment described here, can provide that the filling of each bag 101 is controlled by means of weight or optical sensors.

Some forms of embodiment, described for example using fig. 18, provide, once the bags 101 are full, to bring the presser members 115 closer together so as to bring into contact the walls 114a of the respective tiles 114, closing them at the bottom and consequently closing the mouths 101d of the corresponding bags 101.

In some forms of embodiment, which can be combined with all the forms of embodiment described here, the welding heads 117a are kept reciprocally distanced during the preparation and filling of the bags 101, that is, during the whole packaging process up to the closing of the tiles 114 or mouths 101d of the bags 101.

Fig. 19 is used to describe forms of embodiment in which, once the mouths 101d are closed, the welding heads 117a are moved near to and closed on the mouths 101d of the bags 101, in order to weld them, in accordance with possible implementations of said bag filling and closing step.

After the mouths 101d of the bags 101 have been welded, the presser members 115 are returned to their retracted position, releasing the bags 101 from pressure against the tiles 114 and the bags 101 are discharged from the respective cradles 102.

In some forms of embodiment, the bags 101 can be discharged manually, or alternatively an automatic discharge can be provided, for example by means of the belt conveyor 119 positioned below the cradles 102 and shown in fig. 20. The belt conveyor 119 receives the bags 101 from the cradles 102 due to the distancing of the lower edges 109b of the plates 109 determined by the retraction of the lower linear actuators 110b, which distancing deprives the bags 101 of the support previously supplied by the cradles 102.

Some forms of embodiment, which can be combined with other forms of embodiment described here, concern a method for automatically packaging into bags 101 loose products sterilized exiting from a discharge aperture 15a of a treatment chamber 11 of a sterilizing machine 10, the method comprising:
- feeding one or more bags 101 from at least a bag storage chamber 104 to a respective bag support element or cradle 102, by means of a motorized bag transfer unit 105 which comprises at least a motorized bag removal element 106, inside the bag storage chamber 104, the motorized bag removal element 106 being put in constant contact with a bag 101 and selectively activated to remove the bag 101 from the bag storage chamber 104 and to transfer it to the bag support element 102,
- opening a bag 101 supported by the bag support element 102 to allow the bag 101 to receive loose products sterilized exiting from the discharge aperture 15a,
- sealing a bag 101 filled with the sterilized loose products by means of at least one bag sealing device 117.

Figs. 12a to 16 and 18 to 20 are used to describe forms of embodiment, which can be combined with other forms of embodiment described here, in which the automatic packaging apparatus 100 is configured to automatically package two bags 101 and which includes two bag feed devices 103 configured to feed two bags 101. In this form of embodiment, two bag storage chambers 104, two respective motorized bag transfer units 105 with corresponding two motorized removal rolls 106 are provided. Furthermore, in these forms of embodiment, the automatic packaging apparatus 100 includes two cradles 102 to each receive a respective bag 101 to be filled with sterilized loose products and subsequently heat-welded, two bag opening devices 111, each configured to open a respective bag 101 supported by a corresponding cradle 102 and allow the bag 101 to receive sterilized loose products exiting from a respective discharge aperture 15a of two discharge apertures 15a provided, and two bag sealing devices, for example two heat welding machines 117, each to seal or heat-weld a respective bag 101 filled with said sterilized loose products.

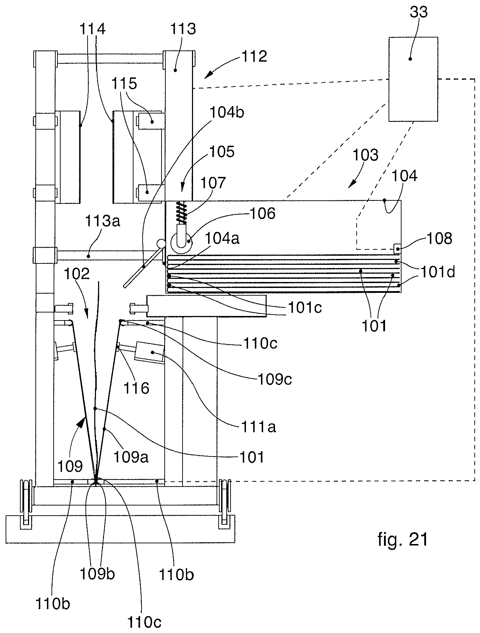

Fig. 21 is used to describe forms of embodiment, which can be combined with other forms of embodiment described here, in which the automatic packaging apparatus 100 is configured to automatically package a bag 101 and which includes a bag packaging device 103 configured to feed a single bag 101 and provided with a single bag storage chamber 104, a single motorized bag transfer unit 105 and a single motorized removal roll 106. Furthermore, in these forms of embodiment, the automatic packaging apparatus 100 includes a single cradle 102 to receive the bag 101 to be filled with sterilized loose products and subsequently heat-welded, a single bag opening device 111 configured to open the bag 101 supported by the cradle 102 and allow the bag 101 to receive sterilized loose products exiting from a single discharge aperture 15a, and a single bag sealing device, for example a single heat welding machine 117, to seal or heat-weld a bag 101 filled with said sterilized loose products.

It is clear that modifications and/or additions of parts may be made to the sterilizing machine 10 and the packaging method as described heretofore, without departing from the field and scope of the present invention. It is also clear that, although the present invention has been described with reference to some specific examples, a person of skill in the art shall certainly be able to achieve many other equivalent forms of sterilizing machine and packaging method, having the characteristics as set forth in the claims and hence all coming within the field of protection defined thereby.

Although the above refers to forms of embodiment of the invention, other forms of embodiment can be provided without departing from the main field of protection, which is defined by the following claims.

## Claims

1. Sterilizing machine for loose products, comprising at least a treatment chamber (11) to sterilize said loose products, **characterized in that** it comprises an apparatus (100) for automatic packaging into bags (101) of the sterilized loose products exiting from a discharge aperture (15a) of said treatment chamber (11), said automatic packaging apparatus (100) comprising:
- at least a bag support element (102) positionable at said discharge aperture (15a);
- at least a bag feed device (103) provided with a bag storage chamber (104) and a motorized bag transfer unit (105) to transfer the bags (101) from said bag storage chamber (104) to the bag support element (102), said motorized bag transfer unit (105) comprising at least a motorized bag removal element (106), inside the bag storage chamber (104), configured to be put in constant contact with a bag (101) and able to be selectively activated to remove said bag (101) from said bag storage chamber (104) and to transfer it to said bag support element (102),
- at least a bag opening device (111) configured to open a bag (101) supported by said bag support element (102) and to allow said bag (101) to receive sterilized loose products exiting from said discharge aperture (15a),
- at least a bag sealing device (117) to seal a bag (101) filled with said sterilized loose products.

2. Sterilizing machine as in claim 1, **characterized in that** said at least one bag feed device (103) comprises a guide element (104b) associated to a bag passage aperture (104a) of said bag storage chamber (104) and configured to guide a bag (101) exiting from said bag passage aperture (104a) toward said bag support element (102).

3. Sterilizing machine as in claim 2, **characterized in that** said automatic packaging apparatus (100) comprises at least a conveying device (112) provided with at least a frame (113) to which one or more deflector members (114) are connected.

4. Sterilizing machine as in claim 3, **characterized in that** said frame (113) comprises a hinging side (113a) and is rotatable with respect to said hinging side (113a) to assume a lowered position, in which said one or more deflector members (114) cooperate with a respective one of said bags (101).

5. Sterilizing machine as in claim 3 or 4, **characterized in that** said conveying device (112) comprises linear actuators (213a) connected to said frame (113) to translate said frame (113) toward a lowered position, in which said one or more deflector members (114) cooperate with a respective one of said bags (101).

6. Sterilizing machine as in any claim from 1 to 5, **characterized in that** said bag support element (102) comprises a pair of mobile plates (109) **and in that** said automatic packaging apparatus (100) comprises at least a lower movement device (110b) and an upper movement device (110c) to respectively move corresponding lower edges (109b) and upper edges (109c) of one of said plates (109) reciprocally closer to and away from the other of said plates (109).

7. Sterilizing machine as in any claim from 1 to 6, **characterized in that** said automatic packaging apparatus (100) comprises an automatic discharge device (119), configured to cooperate with said at least one bag support element (102) to receive and discharge said bag (101) from said at least one bag support element (102).

8. Method for packaging into bags (101) loose products sterilized in a treatment chamber (11) of a sterilizing machine (10) according to any claim from 1 to 7, **characterized in that** it comprises:
- a step of preparing the bags, which provides to transfer each bag (101) automatically from a bag storage chamber (104) to a bag support element (102) and subsequently to position each of said bags (101) in an open condition in correspondence to a discharge aperture (15a) of said treatment chamber (11) in order to receive said loose products,
- a step of filling and closing the bags, which provides to fill said bags (101) with dosed quantities of said loose products exiting from said discharge aperture (15a) and to seal said bags (101) after filling them.

9. Method as in claim 8, **characterized in that,** after sealing, said method provides an automatic discharge of said bags (101) by transferring said bags (101) from said bag support element (102) to an automatic discharge device (119) and subsequently distancing said bags (102) from said support element (102).

10. Method as in claim 8 or 9, **characterized in that,** after opening the bag (101), the method provides to automatically verify the actual and correct positioning of said bag (101) in the bag support element (102) and to position the bag (101) in correspondence to the corresponding discharge aperture (15a) following a positive outcome of said verification.

11. Method as in claim 10, **characterized in that,** following a positive outcome of said verification, the method provides to support and clamp said bag (101) stably in said position in correspondence with the corresponding discharge aperture (15a).

## Patentansprüche

1. Sterilisiermaschine für lose Produkte, umfassend zumindest eine Behandlungskammer (11) zur Sterilisierung der losen Produkte, **dadurch gekennzeichnet, dass** sie eine Vorrichtung (100) zur automatischen Verpackung in Beutel (101) der sterilisierten losen Produkte umfasst, die aus einer Abgabeöffnung (15a) der Behandlungskammer (11) austreten, wobei die automatische Verpackungsvorrichtung (100) umfasst:
- mindestens ein Beuteltragelement (102), das an der Abgabeöffnung (15a) positionierbar ist;
- mindestens eine Beutelzuführvorrichtung (103), die mit einer Beutellagerkammer (104) und mit einer motorisch angetriebenen Beutelüberführeinheit (105) zum Überführen der Beutel (101) von der Beutellagerkammer (104) zum Beuteltragelement (102) versehen ist, worin die motorisch angetriebene Beutelüberführeinheit (105) mindestens ein motorisch angetriebenes Beutelentnahmeelement (106) innerhalb der Beutellagerkammer (104) umfasst, das dafür eingerichtet ist, um mit einem Beutel (101) in ständigem Kontakt gebracht zu werden, und das selektiv betätigt werden kann, um den Beutel (101) von der Beutellagerkammer (104) zu entnehmen und ihn zum Beuteltragelement (102) zu überführen,
- mindestens eine Beutelöffnungsvorrichtung (111), die dafür eingerichtet ist, um einen Beutel (101) zu öffnen, die vom Beuteltragelement (102) getragen wird, und zu ermöglichen, dass der Beutel (101) sterilisierte lose Produkte aufnimmt, die aus der Abgabeöffnung (15a) austreten,
- mindestens eine Beutelversiegelungsvorrichtung (117), um einen Beutel (101) zu versiegeln, die mit den sterilisierten losen Produkten gefüllt ist.

2. Sterilisiermaschine nach Anspruch 1, **dadurch gekennzeichnet, dass** die zumindest eine Beutelzuführvorrichtung (103) ein Führungselement (104b) umfasst, das einer Beuteldurchgangsöffnung (104a) der Beutellagerkammer (104) zugeordnet ist und das dafür eingerichtet ist, um einen Beutel (101), der aus der Beuteldurchgangsöffnung (104a) austritt, zum Beuteltragelement (102) hin zu führen.

3. Sterilisiermaschine nach Anspruch 2, **dadurch gekennzeichnet, dass** die automatische Verpackungsvorrichtung (100) mindestens eine Fördervorrichtung (112) umfasst, die mit zumindest einem Rahmen (113) versehen ist, mit dem ein oder mehrere Ablenkelemente (114) verbunden sind.

4. Sterilisiermaschine nach Anspruch 3, **dadurch gekennzeichnet, dass** der Rahmen (113) eine Anlenkseite (113a) umfasst und relativ zur Anlenkseite (113a) drehbar ist, um eine abgesenkte Stellung einzunehmen, in der das eine oder die mehreren Ablenkelemente (114) jeweils mit einem der Beutel (101) zusammenarbeiten.

5. Sterilisiermaschine nach Anspruch 3 oder 4, **dadurch gekennzeichnet, dass** die Fördervorrichtung (112) Linearaktoren (213a) umfasst, die mit dem Rahmen (113) verbunden sind, um den Rahmen (113) zu einer abgesenkten Stellung hin zu überführen, in der das eine oder die mehreren Ablenkelemente (114) jeweils mit einem der Beutel (101) zusammenarbeiten.

6. Sterilisiermaschine nach einem der Ansprüche 1 bis 5, **dadurch gekennzeichnet, dass** das Beuteltragelement (102) ein Paar bewegliche Platten (109) umfasst, **und dass** die automatische Verpackungsvorrichtung (100) mindestens eine untere Bewegungsvorrichtung (110b) und eine obere Bewegungsvorrichtung (110c) umfasst, um entsprechende untere Kanten (109b) und obere Kanten (109c) der einen Platte (109) jeweils gegenseitig näher zu und weg von der anderen Platte (109) zu bewegen.

7. Sterilisiermaschine nach einem der Ansprüche 1 bis 6, **dadurch gekennzeichnet, dass** die automatische Verpackungsvorrichtung (100) eine automatische Abgabevorrichtung (119) umfasst, die dafür eingerichtet ist, um mit dem zumindest einen Beuteltragelement (102) zusammenzuarbeiten, um den Beutel (101) aus dem zumindest einen Beuteltragelement (102) aufzunehmen und abzugeben.

8. Verfahren zur Verpackung in Beutel (101) von losen Produkten, die in einer Behandlungskammer (11) einer Sterilisiermaschine (10) nach einem der Ansprüche 1 bis 7 sterilisiert werden, **dadurch gekennzeichnet, dass** es umfasst:
- einen Schritt der Vorbereitung der Beutel (101), der vorsieht, jeden Beutel (101) von einer Beutellagerkammer (104) zu einem Beuteltragelement (102) automatisch zu überführen und anschließend jeden Beutel (101) in einem offenen Zustand an einer Abgabeöffnung (15a) der Behandlungskammer (11) zu positionieren, um die losen Produkte aufzunehmen,
- einen Schritt des Füllens und Verschließens der Beutel, der vorsieht, die Beutel (101) mit dosierten Mengen der losen Produkte zu füllen, die aus der Abgabeöffnung (15a) austreten, und die Beutel (101) nach deren Füllung zu versiegeln.

9. Verfahren nach Anspruch 8, **dadurch gekennzeichnet, dass** nach der Füllung das Verfahren eine automatische Abgabe der Beutel (101) vorsieht, indem die Beutel (101) vom Beuteltragelement (102) zu einer automatischen Abgabevorrichtung (119) überführt werden und die Beutel (101) anschließend vom Tragelement (102) beabstandet werden.

10. Verfahren nach Anspruch 8 oder 9, **dadurch gekennzeichnet, dass** nach der Öffnung des Beutels (101) das Verfahren vorsieht, die aktuelle und richtige Positionierung des Beutels (101) im Beuteltragelement (102) automatisch zu prüfen und den Beutel (101) an der entsprechenden Abgabeöffnung (15a) nach einem positiven Ausgang der Überprüfung zu positionieren.

11. Verfahren nach Anspruch 10, **dadurch gekennzeichnet, dass** nach einem positiven Ausgang der Überprüfung das Verfahren vorsieht, den Beutel (101) stabil in der Stellung an der entsprechenden Abgabeöffnung (15a) zu tragen und zu klemmen.

## Revendications

1. Machine de stérilisation pour des produits en vrac, comportant au moins une chambre de traitement (11) pour stériliser lesdits produits en vrac, **caractérisée en ce qu'**elle comporte un appareil (100) pour conditionner automatiquement dans des sacs (101) les produits en vrac stérilisés sortant d'une ouverture d'évacuation (15a) de ladite chambre de traitement (11), ledit appareil de conditionnement automatique (100) comportant :
- au moins un élément de support de sacs (102) pouvant être positionné vers ladite ouverture d'évacuation (15a),
- au moins un dispositif d'alimentation en sacs (103) pourvu d'une chambre de stockage de sacs (104) et d'une unité motorisée de transfert de sacs (105) pour transférer les sacs (101) de ladite chambre de stockage de sacs (104) à l'élément de support de sacs (102), ladite unité motorisée de transfert de sacs (105) comportant au moins un élément motorisé de retrait de sacs (106), à l'intérieur de la chambre de stockage de sacs (104), configuré pour être mis en contact constant avec un sac (101) et apte à être sélectivement activé pour retirer ledit sac (101) de ladite chambre de stockage de sacs (104) et pour le transférer vers ledit élément de support de sacs (102),
- au moins un dispositif d'ouverture de sacs (111) configuré pour ouvrir un sac (101) supporté par ledit élément de support de sacs (102) et pour permettre audit sac (101) de recevoir des produits en vrac stérilisés sortant par ladite ouverture d'évacuation (15a),
- au moins un dispositif de scellage de sacs (117) pour sceller un sac (101) rempli desdits produits en vrac stérilisés.

2. Machine de stérilisation selon la revendication 1, **caractérisée en ce que** ledit au moins un dispositif d'alimentation en sacs (103) comporte un élément de guidage (104b) associé à une ouverture de passage de sacs (104a) de la chambre de stockage de sacs (104) et configuré pour guider un sac (101) sortant par ladite ouverture de passage de sacs (104a) vers ledit élément de support de sacs (102).

3. Machine de stérilisation selon la revendication 2, **caractérisée en ce que** ledit appareil de conditionnement automatique (100) comporte au moins un dispositif de transport (112) pourvu d'au moins un bâti (113) auquel un ou plusieurs éléments déflecteurs (114) sont reliés.

4. Machine de stérilisation selon la revendication 3, **caractérisée en ce que** ledit bâti (113) comporte un côté charnière (113a) et peut tourner par rapport audit côté charnière (113a) pour prendre une position abaissée, dans laquelle ledit ou lesdits éléments déflecteurs (114) coopèrent avec un desdits sacs correspondant (101).

5. Machine de stérilisation selon la revendication 3 ou 4, **caractérisée en ce que** ledit dispositif de transport (112) comporte des actionneurs linéaires (213a) reliés audit bâti (113) pour déplacer en translation ledit bâti (113) vers une position abaissée, dans laquelle ledit ou lesdits éléments déflecteurs (114) coopèrent avec un desdits sacs correspondant (101).

6. Machine de stérilisation selon l'une quelconque des revendications 1 à 5, **caractérisée en ce que** ledit élément de support de sacs (102) comporte une paire de plaques mobiles (109) et **en ce que** ledit appareil de conditionnement automatique (100) comporte au moins un dispositif de déplacement inférieur (110b) et un dispositif de déplacement supérieur (110c) pour rapprocher et éloigner respectivement des bords inférieurs (109b) et des bords supérieurs (109c) correspondants de l'une desdites plaques (109) de l'autre desdites plaques (109).

7. Machine de stérilisation selon l'une quelconque des revendications 1 à 6, **caractérisée en ce que** ledit appareil de conditionnement automatique (100) comporte un dispositif d'évacuation automatique (119), configuré pour coopérer avec ledit au moins un élément de support de sacs (102) pour recevoir et évacuer ledit sac (101) dudit au moins un élément de support de sacs (102).

8. Procédé pour conditionner dans des sacs (101) des produits en vrac stérilisés dans une chambre de traitement (11) d'une machine de stérilisation (10) selon l'une quelconque des revendications 1 à 7, **caractérisé en ce qu'**il comporte :
- une étape de préparation des sacs, qui permet de transférer chaque sac (101) automatiquement depuis une chambre de stockage de sacs (104) vers un élément de support de sacs (102) et ensuite de positionner chacun desdits sacs (101) dans un état ouvert en correspondance avec une ouverture d'évacuation (15a) de ladite chambre de traitement (11) afin de recevoir lesdits produits en vrac,
- une étape de remplissage et de fermeture des sacs, qui permet de remplir lesdits sacs (101) avec des quantités dosées desdits produits en vrac sortant par ladite ouverture d'évacuation (15a) et de sceller lesdits sacs (101) après les avoir remplis.

9. Procédé selon la revendication 8, **caractérisé en ce que**, après scellage, ledit procédé permet une évacuation automatique desdits sacs (101) en transférant lesdits sacs (101) à partir dudit élément de support de sacs (102) vers un dispositif d'évacuation automatique (119) et en éloignant ensuite lesdits sacs (101) dudit élément de support (102).

10. Procédé selon la revendication 8 ou 9, **caractérisé en ce que**, après l'ouverture du sac (101), le procédé permet de vérifier automatiquement le positionnement réel et correct dudit sac (101) dans l'élément de support de sacs (102) et de positionner le sac (101) en correspondance avec l'ouverture d'évacuation (15a) correspondante après un résultat positif de ladite vérification.

11. Procédé selon la revendication 10, **caractérisé en ce que**, après un résultat positif de ladite vérification, le procédé permet de supporter et de serrer ledit sac (101) de manière stable dans ladite position en correspondance avec l'ouverture d'évacuation (15a) correspondante.
